# EUROPÄISCHE PATENTSCHRIFT

(11) **EP 0 941 311 B1**
(45) Veröffentlichungstag und Bekanntmachung des Hinweises auf die Patenterteilung: **28.07.2004**
(21) Anmeldenummer: 97947036.6
(22) Anmeldetag: 30.10.1997
(51) Int. Cl.: C12N 9/52, C12N 15/57, C12P 21/02, A61K 38/46, C07K 14/33

(54) **REKOMBINANTE COLLAGENASE TYP I AUS CLOSTRIDIUM HISTOLYTICUM UND IHRE VERWENDUNG ZUR ISOLIERUNG VON ZELLEN UND ZELLVERBÄNDEN**
TYPE I RECOMBINANT CLOSTRIDIUM HISTOLYTICUM COLLAGENASE AND ITS USE FOR ISOLATING CELLS AND CELL AGGREGATES
COLLAGENASE RECOMBINEE TYPE I PROVENANT DU CLOSTRIDIUM HISTOLYTICUM, ET SON UTILISATION POUR L'ISOLATION DES CELLULES ET DES AGGLOMERATIONS CELLULAIRES

(30) Priorität: 19.11.1996 EP 96118490; 24.01.1997 EP 97101085
(43) Veröffentlichungstag der Anmeldung: 15.09.1999
(73) Patentinhaber: Roche Diagnostics GmbH, 68305 Mannheim (DE)
(72) Erfinder: BURTSCHER, Helmut, D-82392 Habach (DE); AMBROSIUS, Dorothee, D-81477 München (DE); HESSE, Friederike, D-80339 München (DE)
(86) Internationale Anmeldenummer: PCT/EP1997/006002
(87) Internationale Veröffentlichungsnummer: WO 1998/022574

(56) Entgegenhaltungen:
- EP-A- 0 677 586
- WO-A-94/00580
- US-A- 5 177 017
- SCHORPP M ET AL: "STRUCTURAL ORGANIZATION AND CHROMOSOMAL LOCALIZATION OF THE MOUSE COLLAGENASE TYPE I GENE" BIOCHEMICAL JOURNAL, Bd. 308, Nr. 1, 15.Mai 1995, Seiten 211-217, XP000653589
- SMYTH-TEMPLETON N ET AL: "CLONING AND CHARACTERIZATION OF HUMAN TUMOR CELL INTERSTITIAL COLLAGENASE" CANCER RESEARCH, Bd. 50, Nr. 17, 1.September 1990, Seiten 5431-5437, XP000652150

### SEQUENZPROTOKOLL

### (1) ALLGEMEINE ANGABEN:

(i) ANMELDER:
   (A) NAME: BOEHRINGER MANNHEIM GMBH
   (B) STRASSE: Sandhofer Str. 116
   (C) ORT: Mannheim
   (E) LAND: Germany
   (F) POSTLEITZAHL: D-68305
   (G) TELEFON: 08856/60-3446
   (H) TELEFAX: 08856/60-3451
(ii) BEZEICHNUNG DER ERFINDUNG: Rekombinante Collagenase Typ I aus Clostridium histolyticum und ihre Verwendung zur Isolierung von Zellen und Zellverbaenden
(iii) ANZAHL DER SEQUENZEN: 24
(iv) COMPUTER-LESBARE FASSUNG:
   (A) DATENTRÄGER: Floppy disk
   (B) COMPUTER: IBM PC compatible
   (C) BETRIEBSSYSTEM: PC-DOS/MS-DOS
   (D) SOFTWARE: PatentIn Release #1.0, Version #1.30B (EPA)

### (2) ANGABEN ZU SEQ ID NO: 1:

(i) SEQUENZKENNZEICHEN:
   (A) LÄNGE: 4358 Basenpaare
   (B) ART: Nucleotid
   (C) STRANGFORM: Doppelstrang
   (D) TOPOLOGIE: linear
(ii) ART DES MOLEKÜLS: Genom-DNA
(ix) MERKMAL:
   (A) NAME/SCHLÜSSEL: CDS
   (B) LAGE:1332..4358
(xi) SEQUENZBESCHREIBUNG: SEQ ID NO: 1:
   AAGCTTTTAT AATTCTCACT ATACTCTCTA GATGGTATAA TAAGAGCCCA AAATACGAAG    60
   AATATAATAA AATATAATTT AAAAAGGATG ATTTCAATGC GAAAAAGAGT TTTTTTGAAT   120
   ATATTTTTTA TTTTATGTTC CTCAATTTTT TTTATGTCCT GCACAGGAAA ATTTCAGGTT   180
   ATAGATCGGG GTGATGGGGG AGATGAAATT TATTTAAACA AACAAGATGG TGTGAGTTTT   240
   GAGATTCCTA AAGTGTGGGA TAAAAATTAT AAGATTATCA CTTCTAGAGA TAAAAGATAT   300
   GGCAAAAAGT TAACTTTTAA AAAAAAGGAT AAGAAATGCA ACGTTATACT TTTAGAAATA   360
   TGGATTTTGA ATGAGGAATA TTGGAGTGAA TTTAAAGATG TTAGGAAGTT TAAACTTATA   420
   GGTAAAAGCG AAAAAGGCGT AGTAGTTTAT TCAAGAGGTA AATTAGATAG CATATTAGAA   480
   AATAATGGAT TGGACATTAT GCATCATAAA GAAGAGAAAA AGAAAGATAT AGAAAAAATG   540
   TACATTAAAG ATGAAGAAAT TAGCGATAGA ATCAAAATAA TTAGAAATTA ATAAAAAAAT   600
   GAAAATAGAA AAATTCATTT TACTAAAAAT TTATGTTTAC TTTCTATAAC AATCTTTGTA   660
   AACTGTAAAT ACTAATGTAG TATTTTTTAG AAAATAATAA TCTGTTAAAA AGTGTATTTA   720
   GGAACTAAAA ATGAATAAAT TTATAAAAAC TATTTACAAT ATCTAAAATA ATGTATATAA   780
   TTTTTATTAA ATAGATTATT TTGGTATTAA GGGGGTGGTT GAAAGAATAA ACAGAAAATT   840
   GATATAATTC AATAAATAAA ATCTAAAGAG AAATATCTAA GTAATACACA AATCTAATAT   900
   TAAAATCATT TTAATATTAA GAATATTTTG TTAATAGGTA AAGGTTAAAA GGCATTCTAT   960
   TATTAAGGTT AAAAGGTATT AATTATTAAG GGGGATTATC TATGAAAAAA AATATTTTAA  1020
   AGATTCTTAT GGATAGTTAT TCTAAAGAAT CTAAAATTCA AACTGTACGT AGGGTTACGA  1080
   GTGTATCACT TTTAGCGGCA TATCTTACTA TGAATACTTC AAGTTTAGTT TTAGCAAAAC  1140
   CAATAGAAAA TACTAATGAT ACTAGTATAA AAAATGTGGA GAAATTAAGA AATGCTCCAA  1200
   ATGAAGAGAA TAGTAAAAAG GTAGAAGATA GTAAAAATGA TAAGGTAGAA CATGTGAAAA  1260
   ATATAGAAGA GGCAAAGGTT GAGCAAGTTG CACCCGAAGT AAAATCTAAA TCAACTTTAA  1320
   GAAGTGCTTC T ATA GCG AAT ACT AAT TCT GAG AAA TAT GAT TTT GAG TAT    1370
               Ile Ala Asn Thr Asn Ser Glu Lys Tyr Asp Phe Glu Tyr
                1               5                  10
   TTA AAT GGT TTG AGC TAT ACT GAA CTT ACA AAT TTA ATT AAA AAT ATA     1418
   Leu Asn Gly Leu Ser Tyr Thr Glu Leu Thr Asn Leu Ile Lys Asn Ile
       15                  20                  25
   AAG TGG AAT CAA ATT AAT GGT TTA TTT AAT TAT AGT ACA GGT TCT CAA     1466
   Lys Trp Asn Gln Ile Asn Gly Leu Phe Asn Tyr Ser Thr Gly Ser Gln
   30                  35                  40                  45
   AAG TTC TTT GGA GAT AAA AAT CGT GTA CAA GCT ATA ATT AAT GCT TTA     1514
   Lys Phe Phe Gly Asp Lys Asn Arg Val Gln Ala Ile Ile Asn Ala Leu
                   50                  55                  60
   CAA GAA AGT GGA AGA ACT TAC ACT GCA AAT GAT ATG AAG GGT ATA GAA     1562
   Gln Glu Ser Gly Arg Thr Tyr Thr Ala Asn Asp Met Lys Gly Ile Glu
                   65                  70                  75
   ACT TTC ACT GAG GTT TTA AGA GCT GGT TTT TAT TTA GGG TAC TAT AAT    1610
   Thr Phe Thr Glu Val Leu Arg Ala Gly Phe Tyr Leu Gly Tyr Tyr Asn
            80                  85                  90
   GAT GGT TTA TCT TAT TTA AAT GAT AGA AAC TTC CAA GAT AAA TGT ATA    1658
   Asp Gly Leu Ser Tyr Leu Asn Asp Arg Asn Phe Gln Asp Lys Cys Ile
        95                 100                 105
   CCT GCA ATG ATT GCA ATT CAA AAA AAT CCT AAC TTT AAG CTA GGA ACT    1706
   Pro Ala Met Ile Ala Ile Gln Lys Asn Pro Asn Phe Lys Leu Gly Thr
   110                 115                 120                 125
   GCA GTT CAA GAT GAA GTT ATA ACT TCT TTA GGA AAA CTA ATA GGA AAT    1754
   Ala Val Gln Asp Glu Val Ile Thr Ser Leu Gly Lys Leu Ile Gly Asn
                   130                 135                 140
   GCT TCT GCT AAT GCT GAA GTA GTT AAT AAT TGT GTA CCA GTT CTA AAA    1802
   Ala Ser Ala Asn Ala Glu Val Val Asn Asn Cys Val Pro Val Leu Lys
               145                 150                 155
   CAA TTT AGA GAA AAC TTA AAT CAA TAT GCT CCT GAT TAC GTT AAA GGA    1850
   Gln Phe Arg Glu Asn Leu Asn Gln Tyr Ala Pro Asp Tyr Val Lys Gly
           160                 165                 170
   ACA GCT GTA AAT GAA TTA ATT AAA GGT ATT GAA TTC GAT TTT TCT GGT    1898
   Thr Ala Val Asn Glu Leu Ile Lys Gly Ile Glu Phe Asp Phe Ser Gly
       175                 180                 185
   GCT GCA TAT GAA AAA GAT GTT AAG ACA ATG CCT TGG TAT GGA AAA ATT    1946
   Ala Ala Tyr Glu Lys Asp Val Lys Thr Met Pro Trp Tyr Gly Lys Ile
   190                 195                 200                 205
   GAT CCA TTT ATA AAT GAA CTT AAG GCC TTA GGT CTA TAT GGA AAT ATA    1994
   Asp Pro Phe Ile Asn Glu Leu Lys Ala Leu Gly Leu Tyr Gly Asn Ile
                   210                 215                 220
   ACA AGT GCA ACT GAG TGG GCA TCT GAT GTT GGA ATA TAC TAT TTA AGT    2042
   Thr Ser Ala Thr Glu Trp Ala Ser Asp Val Gly Ile Tyr Tyr Leu Ser
               225                 230                 235
   AAA TTC GGG CTT TAC TCA ACT AAC CGA AAT GAC ATA GTA CAG TCA CTT    2090
   Lys Phe Gly Leu Tyr Ser Thr Asn Arg Asn Asp Ile Val Gln Ser Leu
           240                 245                 250
   GAA AAG GCT GTA GAT ATG TAT AAG TAT GGT AAA ATA GCC TTT GTA GCA    2138
   Glu Lys Ala Val Asp Met Tyr Lys Tyr Gly Lys Ile Ala Phe Val Ala
       255                 260                 265
   ATG GAG AGA ATA ACT TGG GAT TAT GAT GGG ATT GGT TCT AAT GGT AAA    2186
   Met Glu Arg Ile Thr Trp Asp Tyr Asp Gly Ile Gly Ser Asn Gly Lys
   270                 275                 280                 285
   AAG GTG GAT CAC GAT AAG TTC TTA GAT GAT GCT GAA AAA CAT TAT CTG    2234
   Lys Val Asp His Asp Lys Phe Leu Asp Asp Ala Glu Lys His Tyr Leu
                   290                 295                 300
   CCA AAG ACA TAT ACT TTT GAT AAT GGA ACC TTT ATT ATA AGA GCA GGG     2282
   Pro Lys Thr Tyr Thr Phe Asp Asn Gly Thr Phe Ile Ile Arg Ala Gly
               305                 310                 315
   GAT AAG GTA TCC GAA GAA AAA ATA AAA AGG CTA TAT TGG GCA TCA AGA     2330
   Asp Lys Val Ser Glu Glu Lys Ile Lys Arg Leu Tyr Trp Ala Ser Arg
           320                 325                 330
   GAA GTG AAG TCT CAA TTC CAT AGA GTA GTT GGC AAT GAT AAA GCT TTA     2378
   Glu Val Lys Ser Gln Phe His Arg Val Val Gly Asn Asp Lys Ala Leu
       335                 340                 345
   GAG GTG GGA AAT GCC GAT GAT GTT TTA ACT ATG AAA ATA TTT AAT AGC     2426
   Glu Val Gly Asn Ala Asp Asp Val Leu Thr Met Lys Ile Phe Asn Ser
   350                 355                 360                 365
   CCA GAA GAA TAT AAA TTT AAT ACC AAT ATA AAT GGT GTA AGT ACT GAT     2474
   Pro Glu Glu Tyr Lys Phe Asn Thr Asn Ile Asn Gly Val Ser Thr Asp
                   370                 375                 380
   AAT GGT GGT CTA TAT ATA GAA CCA AGA GGG ACT TTC TAC ACT TAT GAG     2522
   Asn Gly Gly Leu Tyr Ile Glu Pro Arg Gly Thr Phe Tyr Thr Tyr Glu
               385                 390                 395
   AGA ACA CCT CAA CAA AGT ATA TTT AGT CTT GAA GAA TTG TTT AGA CAT     2570
   Arg Thr Pro Gln Gln Ser Ile Phe Ser Leu Glu Glu Leu Phe Arg His
           400                 405                 410
   GAA TAT ACT CAC TAT TTA CAA GCG AGA TAT CTT GTA GAT GGT TTA TGG     2618
   Glu Tyr Thr His Tyr Leu Gln Ala Arg Tyr Leu Val Asp Gly Leu Trp
       415                 420                 425
   GGG CAA GGT CCA TTT TAT GAA AAA AAT AGA TTA ACT TGG TTT GAT GAA     2666
   Gly Gln Gly Pro Phe Tyr Glu Lys Asn Arg Leu Thr Trp Phe Asp Glu
   430                 435                 440                 445
   GGT ACA GCT GAA TTC TTT GCA GGA TCT ACC CGT ACA TCT GGT GTT TTA     2714
   Gly Thr Ala Glu Phe Phe Ala Gly Ser Thr Arg Thr Ser Gly Val Leu
                   450                 455                 460
   CCA AGA AAA TCA ATA TTA GGA TAT TTG GCT AAG GAT AAA GTA GAT CAT     2762
   Pro Arg Lys Ser Ile Leu Gly Tyr Leu Ala Lys Asp Lys Val Asp His
               465                 470                 475
   AGA TAC TCA TTA AAG AAG ACT CTT AAT TCA GGG TAT GAT GAC AGT GAT     2810
   Arg Tyr Ser Leu Lys Lys Thr Leu Asn Ser Gly Tyr Asp Asp Ser Asp
           480                 485                 490
   TGG ATG TTC TAT AAT TAT GGA TTT GCA GTT GCA CAT TAC CTA TAT GAA     2858
   Trp Met Phe Tyr Asn Tyr Gly Phe Ala Val Ala His Tyr Leu Tyr Glu
       495                 500                 505
   AAA GAT ATG CCT ACA TTT ATT AAG ATG AAT AAA GCT ATA TTG AAT ACA     2906
   Lys Asp Met Pro Thr Phe Ile Lys Met Asn Lys Ala Ile Leu Asn Thr
   510                 515                 520                 525
   GAT GTG AAA TCT TAT GAT GAA ATA ATA AAA AAA TTA AGT GAT GAT GCA     2954
   Asp Val Lys Ser Tyr Asp Glu Ile Ile Lys Lys Leu Ser Asp Asp Ala
                   530                 535                 540
   AAT AAA AAT ACA GAA TAT CAA AAC CAT ATT CAA GAG TTA GTA GAT AAA     3002
   Asn Lys Asn Thr Glu Tyr Gln Asn His Ile Gln Glu Leu Val Asp Lys
               545                 550                 555
   TAT CAA GGA GCT GGA CTA CCT CTA GTA TCA GAT GAT TAC TTA AAA GAT     3050
   Tyr Gln Gly Ala Gly Leu Pro Leu Val Ser Asp Asp Tyr Leu Lys Asp
           560                 565                 570
   CAT GGA TAT AAG AAA GCA TCT GAA GTA TAT TCT GAA ATT TCA AAA GCT     3098
   His Gly Tyr Lys Lys Ala Ser Glu Val Tyr Ser Glu Ile Ser Lys Ala
       575                 580                 585
   GCT TCT CTT ACA AAC ACT AGT GTA ACA GCA GAA AAA TCT CAA TAC TTT     3146
   Ala Ser Leu Thr Asn Thr Ser Val Thr Ala Glu Lys Ser Gln Tyr Phe
   590                 595                 600                 605
   AAC ACA TTC ACT TTA AGA GGA ACT TAT ACA GGT GAA ACT TCT AAA GGT     3194
   Asn Thr Phe Thr Leu Arg Gly Thr Tyr Thr Gly Glu Thr Ser Lys Gly
                   610                 615                 620
   GAA TTT AAA GAT TGG GAT GAA ATG AGT AAA AAA TTA GAT GGA ACT TTG     3242
   Glu Phe Lys Asp Trp Asp Glu Met Ser Lys Lys Leu Asp Gly Thr Leu
               625                 630                 635
   GAG TCC CTT GCT AAA AAT TCT TGG AGT GGA TAC AAA ACC TTA ACA GCA     3290
   Glu Ser Leu Ala Lys Asn Ser Trp Ser Gly Tyr Lys Thr Leu Thr Ala
           640                 645                 650
   TAC TTT ACG AAT TAT AGA GTT ACA AGC GAT AAT AAA GTT CAA TAT GAT     3338
   Tyr Phe Thr Asn Tyr Arg Val Thr Ser Asp Asn Lys Val Gln Tyr Asp
       655                 660                 665
   GTA GTT TTC CAT GGG GTT TTA ACA GAT AAT GGG GAT ATT AGT AAC AAT     3386
   Val Val Phe His Gly Val Leu Thr Asp Asn Gly Asp Ile Ser Asn Asn
   670                 675                 680                 685
   AAG GCT CCA ATA GCA AAG GTA ACT GGA CCA AGC ACT GGT GCT GTA GGA     3434
   Lys Ala Pro Ile Ala Lys Val Thr Gly Pro Ser Thr Gly Ala Val Gly
                   690                 695                 700
   AGA AAT ATT GAA TTT AGT GGA AAA GAT AGT AAA GAT GAA GAT GGT AAA     3482
   Arg Asn Ile Glu Phe Ser Gly Lys Asp Ser Lys Asp Glu Asp Gly Lys
               705                 710                 715
   ATA GTA TCA TAT GAT TGG GAT TTT GGC GAT GGT GCA ACT AGT AGA GGC     3530
   Ile Val Ser Tyr Asp Trp Asp Phe Gly Asp Gly Ala Thr Ser Arg Gly
           720                 725                 730
   AAA AAT TCA GTA CAT GCT TAC AAA AAA GCA GGA ACA TAT AAT GTT ACA     3578
   Lys Asn Ser Val His Ala Tyr Lys Lys Ala Gly Thr Tyr Asn Val Thr
       735                 740                 745
   TTA AAA GTA ACT GAC GAT AAG GGT GCA ACA GCT ACA GAA AGC TTT ACT     3626
   Leu Lys Val Thr Asp Asp Lys Gly Ala Thr Ala Thr Glu Ser Phe Thr
   750                 755                 760                 765
   ATA GAA ATA AAG AAC GAA GAT ACA ACA ACA CCT ATA ACT AAA GAA ATG     3674
   Ile Glu Ile Lys Asn Glu Asp Thr Thr Thr Pro Ile Thr Lys Glu Met
                   770                 775                 780
   GAA CCT AAT GAT GAT ATA AAA GAG GCT AAT GGT CCA ATA GTT GAA GGT     3722
   Glu Pro Asn Asp Asp Ile Lys Glu Ala Asn Gly Pro Ile Val Glu Gly
               785                 790                 795
   GTT ACT GTA AAA GGT GAT TTA AAT GGT TCT GAT GAT GCT GAT ACC TTC     3770
   Val Thr Val Lys Gly Asp Leu Asn Gly Ser Asp Asp Ala Asp Thr Phe
           800                 805                 810
   TAT TTT GAT GTA AAA GAA GAT GGT GAT GTT ACA ATT GAA CTT CCT TAT     3818
   Tyr Phe Asp Val Lys Glu Asp Gly Asp Val Thr Ile Glu Leu Pro Tyr
       815                 820                 825
   TCA GGG TCA TCT AAT TTC ACA TGG TTA GTT TAT AAA GAG GGA GAC GAT     3866
   Ser Gly Ser Ser Asn Phe Thr Trp Leu Val Tyr Lys Glu Gly Asp Asp
   830                 835                 840                 845
   CAA AAT CAT ATT GCA AGT GGT ATA GAT AAG AAT AAC TCA AAA GTT GGA     3914
   Gln Asn His Ile Ala Ser Gly Ile Asp Lys Asn Asn Ser Lys Val Gly
                   850                 855                 860
   ACA TTT AAA GCT ACA AAA GGA AGA CAT TAT GTG TTT ATA TAT AAA CAC     3962
   Thr Phe Lys Ala Thr Lys Gly Arg His Tyr Val Phe Ile Tyr Lys His
               865                 870                 875
   GAT TCT GCT TCA AAT ATA TCC TAT TCT TTA AAC ATA AAA GGA TTA GGT     4010
   Asp Ser Ala Ser Asn Ile Ser Tyr Ser Leu Asn Ile Lys Gly Leu Gly
           880                 885                 890
   AAC GAG AAA TTG AAG GAA AAA GAA AAT AAT GAT TCT TCT GAT AAA GCT     4058
   Asn Glu Lys Leu Lys Glu Lys Glu Asn Asn Asp Ser Ser Asp Lys Ala
       895                 900                 905
   ACA GTT ATA CCA AAT TTC AAT ACC ACT ATG CAA GGT TCA CTT TTA GGT     4106
   Thr Val Ile Pro Asn Phe Asn Thr Thr Met Gln Gly Ser Leu Leu Gly
   910                 915                 920                 925
   GAT GAT TCA AGA GAT TAT TAT TCT TTT GAG GTT AAG GAA GAA GGC GAA     4154
   Asp Asp Ser Arg Asp Tyr Tyr Ser Phe Glu Val Lys Glu Glu Gly Glu
                   930                 935                 940
   GTT AAT ATA GAA CTA GAT AAA AAG GAT GAA TTT GGT GTA ACA TGG ACA     4202
   Val Asn Ile Glu Leu Asp Lys Lys Asp Glu Phe Gly Val Thr Trp Thr
               945                 950                 955
   CTA CAT CCA GAG TCA AAT ATT AAT GAC AGA ATA ACT TAC GGA CAA GTT     4250
   Leu His Pro Glu Ser Asn Ile Asn Asp Arg Ile Thr Tyr Gly Gln Val
           960                 965                 970
   GAT GGT AAT AAG GTA TCT AAT AAA GTT AAA TTA AGA CCA GGA AAA TAT     4298
   Asp Gly Asn Lys Val Ser Asn Lys Val Lys Leu Arg Pro Gly Lys Tyr
       975                 980                 985
   TAT CTA CTT GTT TAT AAA TAC TCA GGA TCA GGA AAC TAT GAG TTA AGG     4346
   Tyr Leu Leu Val Tyr Lys Tyr Ser Gly Ser Gly Asn Tyr Glu Leu Arg
   990                 995                 1000                1005
   GTA AAT AAA TAA                                                     4358
   Val Asn Lys  *

### (2) ANGABEN ZU SEQ ID NO: 2:

(i) SEQUENZKENNZEICHEN:
   (A) LÄNGE: 1009 Aminosäuren
   (B) ART: Aminosäure
   (D) TOPOLOGIE: linear
(ii) ART DES MOLEKÜLS: Protein
(xi) SEQUENZBESCHREIBUNG: SEQ ID NO: 2:
   Ile Ala Asn Thr Asn Ser Glu Lys Tyr Asp Phe Glu Tyr Leu Asn Gly
    1               5                  10                  15
   Leu Ser Tyr Thr Glu Leu Thr Asn Leu Ile Lys Asn Ile Lys Trp Asn
               20                  25                  30
   Gln Ile Asn Gly Leu Phe Asn Tyr Ser Thr Gly Ser Gln Lys Phe Phe
           35                  40                  45
   Gly Asp Lys Asn Arg Val Gln Ala Ile Ile Asn Ala Leu Gln Glu Ser
       50                  55                  60
   Gly Arg Thr Tyr Thr Ala Asn Asp Met Lys Gly Ile Glu Thr Phe Thr
   65                  70                  75                  80
   Glu Val Leu Arg Ala Gly Phe Tyr Leu Gly Tyr Tyr Asn Asp Gly Leu
                   85                  90                  95
   Ser Tyr Leu Asn Asp Arg Asn Phe Gln Asp Lys Cys Ile Pro Ala Met
               100                 105                 110
   Ile Ala Ile Gln Lys Asn Pro Asn Phe Lys Leu Gly Thr Ala Val Gln
           115                 120                 125
   Asp Glu Val Ile Thr Ser Leu Gly Lys Leu Ile Gly Asn Ala Ser Ala
   130 135 140
   Asn Ala Glu Val Val Asn Asn Cys Val Pro Val Leu Lys Gln Phe Arg
   145 150 155 160
   Glu Asn Leu Asn Gln Tyr Ala Pro Asp Tyr Val Lys Gly Thr Ala Val
   165 170 175
   Asn Glu Leu Ile Lys Gly Ile Glu Phe Asp Phe Ser Gly Ala Ala Tyr
   180 185 190
   Glu Lys Asp Val Lys Thr Met Pro Trp Tyr Gly Lys Ile Asp Pro Phe
   195 200 205
   Ile Asn Glu Leu Lys Ala Leu Gly Leu Tyr Gly Asn Ile Thr Ser Ala
   210 215 220
   Thr Glu Trp Ala Ser Asp Val Gly Ile Tyr Tyr Leu Ser Lys Phe Gly
   225 230 235 240
   Leu Tyr Ser Thr Asn Arg Asn Asp Ile Val Gln Ser Leu Glu Lys Ala
   245 250 255
   Val Asp Met Tyr Lys Tyr Gly Lys Ile Ala Phe Val Ala Met Glu Arg
   260 265 270
   Ile Thr Trp Asp Tyr Asp Gly Ile Gly Ser Asn Gly Lys Lys Val Asp
   275 280 285
   His Asp Lys Phe Leu Asp Asp Ala Glu Lys His Tyr Leu Pro Lys Thr
   290 295 300
   Tyr Thr Phe Asp Asn Gly Thr Phe Ile Ile Arg Ala Gly Asp Lys Val
   305 310 315 320
   Ser Glu Glu Lys Ile Lys Arg Leu Tyr Trp Ala Ser Arg Glu Val Lys
   325 330 335
   Ser Gln Phe His Arg Val Val Gly Asn Asp Lys Ala Leu Glu Val Gly
   340 345 350
   Asn Ala Asp Asp Val Leu Thr Met Lys Ile Phe Asn Ser Pro Glu Glu
   355 360 365
   Tyr Lys Phe Asn Thr Asn Ile Asn Gly Val Ser Thr Asp Asn Gly Gly
   370 375 380
   Leu Tyr Ile Glu Pro Arg Gly Thr Phe Tyr Thr Tyr Glu Arg Thr Pro
   385 390 395 400
   Gln Gln Ser Ile Phe Ser Leu Glu Glu Leu Phe Arg His Glu Tyr Thr
   405 410 415
   His Tyr Leu Gln Ala Arg Tyr Leu Val Asp Gly Leu Trp Gly Gln Gly
   420 425 430
   Pro Phe Tyr Glu Lys Asn Arg Leu Thr Trp Phe Asp Glu Gly Thr Ala
   435 440 445
   Glu Phe Phe Ala Gly Ser Thr Arg Thr Ser Gly Val Leu Pro Arg Lys
   450 455 460
   Ser Ile Leu Gly Tyr Leu Ala Lys Asp Lys Val Asp His Arg Tyr Ser
   465 470 475 480
   Leu Lys Lys Thr Leu Asn Ser Gly Tyr Asp Asp Ser Asp Trp Met Phe
   485 490 495
   Tyr Asn Tyr Gly Phe Ala Val Ala His Tyr Leu Tyr Glu Lys Asp Met
   500 505 510
   Pro Thr Phe Ile Lys Met Asn Lys Ala Ile Leu Asn Thr Asp Val Lys
   515 520 525
   Ser Tyr Asp Glu Ile Ile Lys Lys Leu Ser Asp Asp Ala Asn Lys Asn
   530 535 540
   Thr Glu Tyr Gln Asn His Ile Gln Glu Leu Val Asp Lys Tyr Gln Gly
   545 550 555 560
   Ala Gly Leu Pro Leu Val Ser Asp Asp Tyr Leu Lys Asp His Gly Tyr
   565 570 575
   Lys Lys Ala Ser Glu Val Tyr Ser Glu Ile Ser Lys Ala Ala Ser Leu
   580 585 590
   Thr Asn Thr Ser Val Thr Ala Glu Lys Ser Gln Tyr Phe Asn Thr Phe
   595 600 605
   Thr Leu Arg Gly Thr Tyr Thr Gly Glu Thr Ser Lys Gly Glu Phe Lys
   610 615 620
   Asp Trp Asp Glu Met Ser Lys Lys Leu Asp Gly Thr Leu Glu Ser Leu
   625 630 635 640
   Ala Lys Asn Ser Trp Ser Gly Tyr Lys Thr Leu Thr Ala Tyr Phe Thr
   645 650 655
   Asn Tyr Arg Val Thr Ser Asp Asn Lys Val Gln Tyr Asp Val Val Phe
   660 665 670
   His Gly Val Leu Thr Asp Asn Gly Asp Ile Ser Asn Asn Lys Ala Pro
   675 680 685
   Ile Ala Lys Val Thr Gly Pro Ser Thr Gly Ala Val Gly Arg Asn Ile
   690 695 700
   Glu Phe Ser Gly Lys Asp Ser Lys Asp Glu Asp Gly Lys Ile Val Ser
   705 710 715 720
   Tyr Asp Trp Asp Phe Gly Asp Gly Ala Thr Ser Arg Gly Lys Asn Ser
   725 730 735
   Val His Ala Tyr Lys Lys Ala Gly Thr Tyr Asn Val Thr Leu Lys Val
   740 745 750
   Thr Asp Asp Lys Gly Ala Thr Ala Thr Glu Ser Phe Thr Ile Glu Ile
   755 760 765
   Lys Asn Glu Asp Thr Thr Thr Pro Ile Thr Lys Glu Met Glu Pro Asn
   770 775 780
   Asp Asp Ile Lys Glu Ala Asn Gly Pro Ile Val Glu Gly Val Thr Val
   785 790 795 800
   Lys Gly Asp Leu Asn Gly Ser Asp Asp Ala Asp Thr Phe Tyr Phe Asp
   805 810 815
   Val Lys Glu Asp Gly Asp Val Thr Ile Glu Leu Pro Tyr Ser Gly Ser
   820 825 830
   Ser Asn Phe Thr Trp Leu Val Tyr Lys Glu Gly Asp Asp Gln Asn His
   835 840 845
   Ile Ala Ser Gly Ile Asp Lys Asn Asn Ser Lys Val Gly Thr Phe Lys
   850 855 860
   Ala Thr Lys Gly Arg His Tyr Val Phe Ile Tyr Lys His Asp Ser Ala
   865 870 875 880
   Ser Asn Ile Ser Tyr Ser Leu Asn Ile Lys Gly Leu Gly Asn Glu Lys
   885 890 895
   Leu Lys Glu Lys Glu Asn Asn Asp Ser Ser Asp Lys Ala Thr Val Ile
   900 905 910
   Pro Asn Phe Asn Thr Thr Met Gln Gly Ser Leu Leu Gly Asp Asp Ser
   915 920 925
   Arg Asp Tyr Tyr Ser Phe Glu Val Lys Glu Glu Gly Glu Val Asn Ile
   930 935 940
   Glu Leu Asp Lys Lys Asp Glu Phe Gly Val Thr Trp Thr Leu His Pro
   945 950 955 960
   Glu Ser Asn Ile Asn Asp Arg Ile Thr Tyr Gly Gln Val Asp Gly Asn
   965 970 975
   Lys Val Ser Asn Lys Val Lys Leu Arg Pro Gly Lys Tyr Tyr Leu Leu
   980 985 990
   Val Tyr Lys Tyr Ser Gly Ser Gly Asn Tyr Glu Leu Arg Val Asn Lys
           995                 1000                1005
   *

### (2) ANGABEN ZU SEQ ID NO: 3:

(i) SEQUENZKENNZEICHEN:
   (A) LÄNGE: 110 Aminosäuren
   (B) ART: Aminosäure
   (C) STRANGFORM: Einzelstrang
   (D) TOPOLOGIE: linear
(ii) ART DES MOLEKÜLS: Peptid
(xi) SEQUENZBESCHREIBUNG: SEQ ID NO: 3:
   Met Lys Lys Asn Ile Leu Lys Ile Leu Met Asp Ser Tyr Ser Lys Glu
   1               5                   10                  15
   Ser Lys Ile Gln Thr Val Arg Arg Val Thr Ser Val Ser Leu Leu Ala
               20                  25                  30
   Ala Tyr Leu Thr Met Asn Thr Ser Ser Leu Val Leu Ala Lys Pro Ile
           35                  40                  45
   Glu Asn Thr Asn Asp Thr Ser Ile Lys Asn Val Glu Lys Leu Arg Asn
       50                  55                  60
   Ala Pro Asn Glu Glu Asn Ser Lys Lys Val Glu Asp Ser Lys Asn Asp
   65                  70                  75                  80
   Lys Val Glu His Val Lys Asn Ile Glu Glu Ala Lys Val Glu Gln Val
                   85                  90                  95
   Ala Pro Glu Val Lys Ser Lys Ser Thr Leu Arg Ser Ala Ser
               100                 105                 110

### (2) ANGABEN ZU SEQ ID NO: 4:

(i) SEQUENZKENNZEICHEN:
   (A) LÄNGE: 7 Aminosäuren
   (B) ART: Aminosäure
   (C) STRANGFORM: Einzelstrang
   (D) TOPOLOGIE: linear
(ii) ART DES MOLEKÜLS: Peptid
(xi) SEQUENZBESCHREIBUNG: SEQ ID NO: 4:
   Phe Asn Ser Pro Glu Glu Tyr
   1 5

### (2) ANGABEN ZU SEQ ID NO: 5:

(i) SEQUENZKENNZEICHEN:
   (A) LÄNGE: 7 Aminosäuren
   (B) ART: Aminosäure
   (C) STRANGFORM: Einzelstrang
   (D) TOPOLOGIE: linear
(ii) ART DES MOLEKÜLS: Peptid
(xi) SEQUENZBESCHREIBUNG: SEQ ID NO: 5:
   Glu Asp Gly Asp Val Thr Ile
   1 5

### (2) ANGABEN ZU SEQ ID NO: 6:

(i) SEQUENZKENNZEICHEN:
   (A) LÄNGE: 21 Basenpaare
   (B) ART: Nucleotid
   (C) STRANGFORM: Einzelstrang
   (D) TOPOLOGIE: linear
(ii) ART DES MOLEKÜLS: other nucleic acid
   (A) BESCHREIBUNG: /desc = "Primer"
(xi) SEQUENZBESCHREIBUNG: SEQ ID NO: 6:
   TTYAAYWSNC CNGARGARTA Y 21

### (2) ANGABEN ZU SEQ ID NO: 7:

(i) SEQUENZKENNZEICHEN:
   (A) LÄNGE: 21 Basenpaare
   (B) ART: Nucleotid
   (C) STRANGFORM: Einzelstrang
   (D) TOPOLOGIE: linear
(ii) ART DES MOLEKÜLS: other nucleic acid
   (A) BESCHREIBUNG: /desc = "Primer"
(xi) SEQUENZBESCHREIBUNG: SEQ ID NO: 7:
   RTAYTCYTCN GGNSWRTTRA A                                    21

### (2) ANGABEN ZU SEQ ID NO: 8:

(i) SEQUENZKENNZEICHEN:
   (A) LÄNGE: 21 Basenpaare
   (B) ART: Nucleotid
   (C) STRANGFORM: Einzelstrang
   (D) TOPOLOGIE: linear
(ii) ART DES MOLEKÜLS: other nucleic acid
   (A) BESCHREIBUNG: /desc = "Primer"
(xi) SEQUENZBESCHREIBUNG: SEQ ID NO: 8:
   GARGAYGGNG AYGTNACNAT T                                    21

### (2) ANGABEN ZU SEQ ID NO: 9:

(i) SEQUENZKENNZEICHEN:
   (A) LÄNGE: 21 Basenpaare
   (B) ART: Nucleotid
   (C) STRANGFORM: Einzelstrang
   (D) TOPOLOGIE: linear
(ii) ART DES MOLEKÜLS: other nucleic acid
   (A) BESCHREIBUNG: /desc = "Primer"
(xi) SEQUENZBESCHREIBUNG: SEQ ID NO: 9:
   DATNGTNACR TCNCCRTCYT C                                    21

### (2) ANGABEN ZU SEQ ID NO: 10:

(i) SEQUENZKENNZEICHEN:
   (A) LÄNGE: 7 Aminosäuren
   (B) ART: Aminosäure
   (C) STRANGFORM: Einzelstrang
   (D) TOPOLOGIE: linear
(ii) ART DES MOLEKÜLS: Peptid
(xi) SEQUENZBESCHREIBUNG: SEQ ID NO: 10:
   Asp Trp Asp Glu Met Ser Lys
   1               5

### (2) ANGABEN ZU SEQ ID NO: 11:

(i) SEQUENZKENNZEICHEN:
   (A) LÄNGE: 18 Aminosäuren
   (B) ART: Aminosäure
   (C) STRANGFORM: Einzelstrang
   (D) TOPOLOGIE: linear
(ii) ART DES MOLEKÜLS: Peptid
(xi) SEQUENZBESCHREIBUNG: SEQ ID NO: 11:
   Val Thr Gly Pro Ser Thr Gly Ala Val Gly Arg Asn Ile Glu Phe Ser
   1               5                   10                  15
   Gly Lys

### (2) ANGABEN ZU SEQ ID NO: 12:

(i) SEQUENZKENNZEICHEN:
   (A) LÄNGE: 14 Aminosäuren
   (B) ART: Aminosäure
   (C) STRANGFORM: Einzelstrang
   (D) TOPOLOGIE: linear
(ii) ART DES MOLEKÜLS: Peptid
(xi) SEQUENZBESCHREIBUNG: SEQ ID NO: 12:
   Asn Thr Glu Tyr Gln Asn His Ile Gln Glu Leu Val Asp Lys
   1               5                   10

### (2) ANGABEN ZU SEQ ID NO: 13:

(i) SEQUENZKENNZEICHEN:
   (A) LÄNGE: 22 Aminosäuren
   (B) ART: Aminosäure
   (C) STRANGFORM: Einzelstrang
   (D) TOPOLOGIE: linear
(ii) ART DES MOLEKÜLS: Peptid
(xi) SEQUENZBESCHREIBUNG: SEQ ID NO: 13:
   Glu Asp Gly Asp Val Thr Ile Glu Leu Pro Tyr Ser Gly Ser Ser Asn
   1               5                   10                  15
   Phe Thr Trp Leu Val Tyr
               20

### (2) ANGABEN ZU SEQ ID NO: 14:

(i) SEQUENZKENNZEICHEN:
   (A) LÄNGE: 15 Aminosäuren
   (B) ART: Aminosäure
   (C) STRANGFORM: Einzelstrang
   (D) TOPOLOGIE: linear
(ii) ART DES MOLEKÜLS: Peptid
(xi) SEQUENZBESCHREIBUNG: SEQ ID NO: 14:
   Gln Phe Arg Glu Asn Leu Asn Gln Tyr Ala Pro Asp Tyr Val Lys
   1               5                   10                  15

### (2) ANGABEN ZU SEQ ID NO: 15:

(i) SEQUENZKENNZEICHEN:
   (A) LÄNGE: 19 Basenpaare
   (B) ART: Nucleotid
   (C) STRANGFORM: Einzelstrang
   (D) TOPOLOGIE: linear
(ii) ART DES MOLEKÜLS: other nucleic acid
   (A) BESCHREIBUNG: /desc = "Primer"
(xi) SEQUENZBESCHREIBUNG: SEQ ID NO: 15:
   AGACAATGCC TTGGTATGG 19

### (2) ANGABEN ZU SEQ ID NO: 16:

(i) SEQUENZKENNZEICHEN:
   (A) LÄNGE: 21 Basenpaare
   (B) ART: Nucleotid
   (C) STRANGFORM: Einzelstrang
   (D) TOPOLOGIE: linear
(ii) ART DES MOLEKÜLS: other nucleic acid
   (A) BESCHREIBUNG: /desc = "Primer"
(xi) SEQUENZBESCHREIBUNG: SEQ ID NO: 16:
   GTGAAAGTTT CTATACCCTT C 21

### (2) ANGABEN ZU SEQ ID NO: 17:

(i) SEQUENZKENNZEICHEN:
   (A) LÄNGE: 20 Basenpaare
   (B) ART: Nucleotid
   (C) STRANGFORM: Einzelstrang
   (D) TOPOLOGIE: linear
(ii) ART DES MOLEKÜLS: other nucleic acid
   (A) BESCHREIBUNG: /desc = "Primer"
(xi) SEQUENZBESCHREIBUNG: SEQ ID NO: 17:
   GTACAGGTTC TCAAAAGTTC 20

### (2) ANGABEN ZU SEQ ID NO: 18:

(i) SEQUENZKENNZEICHEN:
   (A) LÄNGE: 20 Basenpaare
   (B) ART: Nucleotid
   (C) STRANGFORM: Einzelstrang
   (D) TOPOLOGIE: linear
(ii) ART DES MOLEKÜLS: other nucleic acid
   (A) BESCHREIBUNG: /desc = "Primer"
(xi) SEQUENZBESCHREIBUNG: SEQ ID NO: 18:
   GTTCCTTTAA CGTAATCAGG 20

### (2) ANGABEN ZU SEQ ID NO: 19:

(i) SEQUENZKENNZEICHEN:
   (A) LÄNGE: 8 Aminosäuren
   (B) ART: Aminosäure
   (C) STRANGFORM: Einzelstrang
   (D) TOPOLOGIE: linear
(ii) ART DES MOLEKÜLS: Peptid
(xi) SEQUENZBESCHREIBUNG: SEQ ID NO: 19:
   Ile Ala Asn Thr Asn Ser Glu Lys
   1               5

### (2) ANGABEN ZU SEQ ID NO: 20:

(i) SEQUENZKENNZEICHEN:
   (A) LÄNGE: 18 Aminosäuren
   (B) ART: Aminosäure
   (C) STRANGFORM: Einzelstrang
   (D) TOPOLOGIE: linear
(ii) ART DES MOLEKÜLS: Peptid
(xi) SEQUENZBESCHREIBUNG: SEQ ID NO: 20:
   Met Thr Met Ile Thr Asn Ser Arg Ala Met Lys Lys Asn Ile Leu Lys
   1               5                   10                  15
   Ile Leu

### (2) ANGABEN ZU SEQ ID NO: 21:

(i) SEQUENZKENNZEICHEN:
   (A) LÄNGE: 18 Aminosäuren
   (B) ART: Aminosäure
   (C) STRANGFORM: Einzelstrang
   (D) TOPOLOGIE: linear
(ii) ART DES MOLEKÜLS: Peptid
(xi) SEQUENZBESCHREIBUNG: SEQ ID NO: 21:
   Met Thr Met Ile Thr Asn Ser Arg Ala Met Asp Ser Tyr Ser Lys Glu
   1               5                   10                  15
   Ser Lys

### (2) ANGABEN ZU SEQ ID NO: 22:

(i) SEQUENZKENNZEICHEN:
   (A) LÄNGE: 18 Aminosäuren
   (B) ART: Aminosäure
   (C) STRANGFORM: Einzelstrang
   (D) TOPOLOGIE: linear
(ii) ART DES MOLEKÜLS: Peptid
(xi) SEQUENZBESCHREIBUNG: SEQ ID NO: 22:
   Met Thr Met Ile Thr Asn Ser Arg Ala Met Lys Asn Ile Glu Glu Ala
   1               5                   10                  15
   Lys Val

### (2) ANGABEN ZU SEQ ID NO: 23:

(i) SEQUENZKENNZEICHEN:
   (A) LÄNGE: 42 Basenpaare
   (B) ART: Nucleotid
   (C) STRANGFORM: Einzelstrang
   (D) TOPOLOGIE: linear
(ii) ART DES MOLEKÜLS: other nucleic acid
   (A) BESCHREIBUNG: /desc = "Primer"
(xi) SEQUENZBESCHREIBUNG: SEQ ID NO: 23:
   TTCCCAATTG CCCGGGCTAT GAAAAATATA GAAGAGGCAA AG                   42

### (2) ANGABEN ZU SEQ ID NO: 24:

(i) SEQUENZKENNZEICHEN:
   (A) LÄNGE: 8 Aminosäuren
   (B) ART: Aminosäure
   (C) STRANGFORM: Einzelstrang
   (D) TOPOLOGIE: linear
(ii) ART DES MOLEKÜLS: Peptid
(xi) SEQUENZBESCHREIBUNG: SEQ ID NO: 24:
   Ala Tyr Leu Thr Met Asn Thr Ser
   1               5

## Beschreibung

Gegenstand der Erfindung ist eine rekombinante Collagenase Typ I (CHC I) aus Clostridium histolyticum und ihre Verwendung zur Isolierung von Zellen und Zellverbänden.

Bakterielle Collagenasen, z.B. aus Clostridium histolyticum, werden zum Verdau von Geweben und zur Gewinnung von Einzelzellen oder Zellverbänden (z.B. Inseln) verwendet (Inseln: Sutton et al., Transplantation 42 (1986) 689 - 691; Leber: Quibel et al., Anal. Biochem. 154 (1986) 26 - 28; Knochen: Hefley et al., J. Bone Mineral Res. 2 (1987) 505 - 516; Nabelschnur: Holzinger et al., Immunol. Lett. 35 (1993) 109 - 118). Aus Clostridium histolyticum sind zwei verschiedene Collagenase-Typen bekannt (M.F. French et al., J. Protein Chemistry 11 (1992) 83 - 97). Die Isolierung und Reinigung von Collagenasen aus Clostridium histolyticum ist beispielsweise beschrieben in E.L. Angleton und H.E. van Wart, Biochemistry 27 (1988) 7413 - 7418 und 7406 - 7412.

Collagenasen I wurden von M.D. Bond und H.E. Van Wart, Biochemistry 23 (1984) 3077 - 3085 und 3085 - 3091 sowie von H.E. Van Wart, Biochemistry 24 (1985) 6520 - 6526 isoliert und beschrieben. Danach sind als Collagenasen I die Collagenasen α (MG 68 kD), β (MG 115 kD), γ (MG 79 kD) und η (MG 130 kD) bekannt. Collagenasen I und Collagenasen II unterscheiden sich in ihrer relativen Aktivität gegen Collagen und gegen synthetische Peptide. Collagenasen I haben höhere Aktivitäten gegenüber Collagen und Gelatine und geringere Aktivitäten gegenüber den kurzkettigen Peptiden als Collagenase II (Bond und Wart; Biochemistry 23 (1984) 3085 - 3091).

Weitere Collagenasen sind beschrieben in den US-Patenten 5,418,157 und 5,177,011 mit einem Molekulargewicht von 68 kD. Die WO 91/14447 beschreibt ebenfalls eine Collagenase mit einem Molekulargewicht von 68 kD. In der WO 94/00580 wird eine rekombinante Collagenase mit einem Molekulargewicht von ca. 110 kD beschrieben und eine Sequenz hierfür angegeben. Über deren Spezifität und insbesondere, ob es sich um eine Collagenase I oder II handelt, wird in der WO 94/00580 nichts ausgesagt. Neben einer Collagenase mit dem Molekulargewicht von 110 kD wird noch eine 125 kD-Collagenase erwähnt, die ebenfalls rekombinant herstellbar sein soll. Nähere Angaben zu dieser Collagenase sind jedoch in der WO 94/00580 nicht enthalten, siehe auch die Patentdokumente US 5 177 017 und EP-A-0 677 586.

Aufgabe der vorliegenden Erfindung ist die Bereitstellung einer hochaktiven stabilen Collagenase Class I aus Clostridium histolyticum (CHC I).

Die Aufgabe wird gelöst durch ein Verfahren zur Herstellung eines Polypeptids, welches die Eigenschaften einer CHC I aus Clostridium histolyticum aufweist, eine vorgegebene Aminosäurezusammensetzung besitzt und erhältlich ist durch Expression einer exogenen Nukleinsäure in prokaryontischen oder eukaryontischen Wirtszellen und Isolierung des gewünschten Polypeptids, wobei die Nukleinsäure für ein Polypeptid der Sequenz ID NO:2 oder ein Polypeptid, welches N-terminal um eine oder mehrere Aminosäuren der Sequenz ID NO:3 verlängert ist, codiert.

Es hat sich überraschenderweise gezeigt, daß eine erfindungsgemäße CHC I eine hohe Collagenase Class I-Aktivität zeigt und sehr stabil ist. Bevorzugt ist eine CHC I der Aminosäuresequenz SEQ ID NO:2 und eine CHC I, welche N-terminal um eine oder mehrere Aminosäuren der SEQ ID NO:3 verlängert ist.

Die erfindungsgemäße CHC I ist ein hochreines Enzym, welches in größeren Mengen hergestellt werden kann. Die erfindungsgemäße CHC I enthält keine Verunreinigungen durch andere clostridiale Enzyme und ist frei von Toxinen.

Die erfindungsgemäße CHC I ist insbesondere zur Isolierung von Zellen aus Geweben von Säugetieren, vorzugsweise aus Humangewebe, für zelltherapeutische Anwendung (Transplantation, Immuntherapie) und für die gentherapeutische Anwendung in den Geweben: z.B. Pankreas, Leber, Knochen, Knorpel, Haut, Gehirn und Nervengewebe, Fettmuskel, Herz, Endothel, Niere, solide Tumoren und zur Reinigung von Ulcera geeignet.

Darüber hinaus ist dieses hochreine Enzym (vorzugsweise im Gemisch mit anderen hochreinen Enzymen (wie Collagenase II und neutrale Protease) für die Isolierung von Zellen, deren Oberflächenmoleküle/Marker (Antigene) nicht verändert werden sollen, besonders geeignet. Bevorzugte Anwendungen hierfür sind beispielsweise die Dissoziation von soliden Tumoren aller Art in vitro (z. B. Kolon, Brust etc.) für die adoptive Immuntherapie und zur allgemeinen Diagnostik, wie z. B. zum Nachweis von seltenen Zellen aus Geweben und soliden Tumoren über spezifische Oberflächenmarker/-Moleküle.

Die Herstellung der rekombinanten CHC I kann nach den dem Fachmann geläufigen Methoden erfolgen.

Dazu wird zunächst ein DNA-Molekül hergestellt, welches in der Lage ist, ein Protein zu produzieren, welches die Aktivität von CHC I besitzt. Die DNA-Sequenz wird in einen Vektor kloniert, der in eine Wirtszelle transferiert werden kann und dort replizierbar ist. Ein derartiger Vektor enthält zusätzlich zur CHC I-Sequenz Promotor/Operator-Elemente, die zur Expression der DNA-Sequenz erforderlich sind. Dieser Vektor, der die CHC I-Sequenz und die Promotor/Operator-Elemente enthält, wird in eine Wirtszelle transferiert, die in der Lage ist, die DNA von CHC I zu exprimieren. Die Wirtszelle wird unter Bedingungen, die zur Amplifikation des Vektors geeignet sind, kultiviert und CHC I gewonnen. Dabei wird durch geeignete Maßnahmen sichergestellt, daß das Protein eine aktive tertiäre Struktur einnehmen kann, in der es CHC I-Eigenschaften zeigt.

Nukleinsäuresequenz und Proteinsequenz können in üblichem Umfang modifiziert sein. Derartige Modifikationen sind beispielsweise:
- Veränderung der Nukleinsäure, um verschiedene Erkennungssequenzen von Restriktionsenzymen zur Erleichterung der Schritte der Ligation, Klonierung und Mutagenese einzuführen
- Veränderung der Nukleinsäure zum Einbau von bevorzugten Codons für die Wirtszelle
- Ergänzung der Nukleinsäure um zusätzliche Operator-Elemente, um die Expression in der Wirtszelle zu optimieren.
- Austausch oder Deletion von Aminosäuren unter Beibehaltung der Basizität und der Raumstruktur der CHC I. Vorteilhaft ist, wenn die ursprüngliche Aminosäuresequenz zu 85 % oder mehr, vorzugsweise zu 90 % oder mehr, erhalten bleibt.

Ein weiterer Gegenstand der Erfindung ist ein Polypeptid mit der Eigenschaft einer Collagenase Class I aus Clostridium histolyticum mit der Aminosäuresequenz gemäß SEQ ID NO:2 oder ein um eine oder mehrere Aminosäuren der SEQ ID NO:3 N-terminal verlängertes Polypeptid. Ein weiterer Gegenstand der Erfindung ist eine für ein derartiges Protein codierende Nukleinsäure.

Die Herstellung des Proteins erfolgt vorzugsweise rekombinant in Mikroorganismen, insbesondere in Prokaryonten, und dort in E. coli.

Die hierfür geeigneten Expressionsvektoren müssen einen Promotor enthalten, der die Expression des Proteins im Wirtsorganismus erlaubt. Derartige Promotoren sind dem Fachmann bekannt und sind beispielsweise lac Promotor (Chang et al., Nature 198 (1977) 1056), trp Promotor (Goeddel et al., Nuc. Acids Res. 8 (1980) 4057), λ_{PL} Promotor (Shimatake et al., Nature 292 (1981) 128) und T5 Promotor (US-Patent 4,689,406). Ebenfalls geeignet sind synthetische Promotoren wie beispielsweise tac Promotor (US-Patent 4,551,433). Ebenso geeignet sind gekoppelte Promotorsysteme wie beispielsweise T7-RNA-Polymerase/Promotorsystem (Studier et al., J. Mol. Biol. 189 (1986) 113). Ebenso geeignet sind hybride Promotoren aus einem Bacteriophagen-Promotor und der Operator-Region des Mikroorganismus (EP-A 0 267 851). Zusätzlich zum Promotor ist eine effektive Ribosomenbindungsstelle erforderlich. Für E. coli wird diese Ribosomenbindungsstelle als Shine-Dalgarno (SD)-Sequenz bezeichnet (Sambrook et al., "Expression of cloned genes in E. coli" in Molecular Cloning: A laboratory manual (1989) Cold Spring Harbor Laboratory Press, New York, USA).

Zur Verbesserung der Expression ist es möglich, das Protein als Fusionsprotein zu exprimieren. In diesem Fall wird üblicherweise eine DNA-Sequenz, welche für den N-terminalen Teil eines endogenen bakteriellen Proteins oder ein anderes stabiles Protein codiert, an das 5'-Ende der für CHC I codierenden Sequenz fusioniert. Beispiele hierfür sind beispielsweise lacZ (Phillips and Silhavy, Nature 344 (1990) 882 - 884), trpE (Yansura , Meth. Enzymol. 185 (1990) 161 - 166).

Nach Expression des Vektors, vorzugsweise eines biologisch funktionellen Plasmids oder eines viralen Vektors, werden die Fusionsproteine vorzugsweise mit Enzymen (z.B. Faktor Xa) gespalten (Nagai et al., Nature 309 (1984) 810). Weitere Beispiele für die Spaltstellen die IgA-Protease-Spaltstelle (WO 91/11520, EP-A 0 495 398) und die Ubiquitin-Spaltstelle (Miller et al., Bio/Technology 7 (1989) 698).

Die auf diese Weise in Bakterien exprimierten Proteine werden durch Aufschluß der Bakterien und Proteinisolierung in üblicher Weise gewonnen.

In einer weiteren Ausführungsform ist es möglich, die Proteine als aktive Proteine aus den Mikroorganismen zu sezernieren. Hierzu wird vorzugsweise ein Fusionsprodukt verwendet, welches aus der Signalsequenz, die für die Sekretion von Proteinen in den verwendeten Wirtsorganismen geeignet ist, und der Nukleinsäure, welche für das Protein codiert, besteht. Das Protein wird dabei entweder in das Medium (bei grampositiven Bakterien) oder in den periplasmatischen Raum (bei gramnegativen Bakterien) sezerniert. Zwischen der Signalsequenz und der für CHC I codierenden Sequenz ist zweckmäßig eine Spaltstelle angebracht, die entweder bei der Prozessierung oder in einem zusätzlichen Schritt die Abspaltung des Proteins erlaubt. Derartige Signalsequenzen stammen beispielsweise von ompA (Ghrayeb et al., EMBO J. 3 (1984) 2437), phoA (Oka et al., Proc. Natl. Acad. Sci. USA 82 (1985) 7212).

Zusätzlich enthalten die Vektoren noch Terminatoren. Terminatoren sind DNA-Sequenzen, die das Ende eines Transkriptionsvorganges signalisieren. Sie zeichnen sich meist durch zwei strukturelle Eigenarten aus: eine umgekehrt repetitive G/C-reiche Region, die intramolekular eine Doppelhelix bilden kann, sowie eine Anzahl von U(bzw. T)-Resten. Beispiele sind der Haupt-Terminator in der DNA des Phagen fd (Beck und Zink, Gene 16 (1981) 35-58) sowie rrnB (Brosius et al., J. Mol. Biol. 148 (1981) 107 - 127).

Zusätzlich enthalten die Expressionsvektoren üblicherweise einen selektierbaren Marker, um transformierte Zellen zu selektieren. Derartige selektierbare Marker sind beispielsweise die Resistenzgene für Ampicillin, Chloramphenicol, Erythromycin, Kanamycin, Neomycin und Tetracyclin (Davies et al., Ann. Rev. Microbiol. 32 (1978) 469). Ebenso geeignete selektierbare Marker sind die Gene für essentielle Substanzen der Biosynthese von für die Zelle notwendigen Stoffen wie z.B. Histidin, Tryptophan und Leucin.

Es sind eine Vielzahl von geeigneten bakteriellen Vektoren bekannt. Beispielsweise sind für die folgenden Bakterien Vektoren beschrieben: Bacillus subtilis (Palva et al., Proc. Natl. Acad. Sci. USA 79 (1982) 5582), E. coli (Aman et al., Gene 40 (1985) 183; Studier et al., J. Mol. Biol. 189 (1986) 113), Streptococcus cremoris (Powell et al., Appl. Environ. Microbiol. 54 (1988) 655), Streptococcus lividans und Streptomyces lividans (US-Patent 4,747,056).

Weitere gentechnologische Verfahren zur Herstellung und Expression von geeigneten Vektoren sind in J. Sambrook et al., Molecular Cloning: a laboratory manual (1989), Cold Spring Harbor Laboratory Press, New York, N.Y. beschrieben.

Eine Expression von rekombinanter CHC I ist außer in prokaryontischen Mikroorganismen auch in Eukaryonten (wie beispielsweise CHO-Zellen, Hefe oder Insektenzellen) möglich. Als eukaryontisches Expressionssystem wird das Hefesystem oder Insektenzellen bevorzugt. Die Expression in Hefe kann über drei Arten von Hefevektoren (integrierende YI_{P} (yeast integrating plasnüds)-Vektoren, replizierende YR_{P} (yeast replicon plasmids)-Vektoren und episomale YE_{P} (yeast episomal plasmids)-Vektoren erfolgen. Näheres hierzu ist beispielsweise in S.M. Kingsman et al, Tibtech 5 (1987) 53- 57 beschrieben.

Ein weiterer Gegenstand der Erfindung ist ein Verfahren zur Auflösung von Zellgewebe und Freisetzung von darin enthaltenen Zellen oder Zellverbänden durch Inkubation des Zellgewebes mit einer erfindungsgemäßen Collagenase Class I aus Clostridium histolyticum, bis zur Freisetzung der Zellen oder Zellverbände im gewünschten Umfang und Abtrennung der Zellen oder der Zellverbände von den Zellgewebeanteilen. Die Abtrennung der Zellen oder Zellverbände von den Zellgewebsanteilen erfolgt bevorzugt durch Zentrifugation mit einem Dichtegradienten.

Eine Isolierung von Zellen bzw. Zellverbänden aus Geweben (z.B Pankreas, Leber, Haut, Endothel, Nabelschnur, Knochen, Tumorgewebe) erfolgt im allgemeinen durch Inkubation von Organen, Organteilen oder Geweben mit Enzymen, die die umgebende extrazelluläre Bindegewebe-Matrix auflösen (Inseln: Sutton et al., Transplantation 42 (1986) 689 - 691; Leber: Quibel et al., Anal. Biochem. 154 (1986) 26 - 28; Knochen: Hefley et al., J. Bone Mineral Res. 2 (1987) 505 - 516; Nabelschnur: Holzinger et al., Immunol. Lett. 35 (1993) 109 - 118). Derartig isolierte Tumorzellen können vorteilhaft zur Tumorvakzinierung und/oder adoptiven Immuntherapie verwendet werden. Eine Gewebsdesintegration kann auch durch Perfusion des gesamten Organs (Ricordi et al, Diabetes 37 (1988) 413 - 420) mit Enzymlösung erfolgen. Entscheidend neben der Zusammensetzung des Enzymgemisches ist dabei die Dauer, der pH-Wert und die Temperatur des Verdaus sowie auch die mechanische Einwirkung, z.B. durch Schütteln und Zusatz von Metallkugeln. Da extrazelluläre Bindegewebsmatrix oft einen hohen Collagen-Anteil aufweist, kommt den Collagenasen eine besondere Rolle zu (Wolters, Hormone and Metabolic Research 26 (1994) 80).

Vorzugsweise wird das erfindungsgemäße Verfahren zur Isolierung von Inseln oder Inselzellen aus Pankreasgewebe verwendet.

Daneben kann der Zusatz weiterer Enzyme, wie Proteinasen (z. B. neutrale Protease, vgl. Bsp. 5, oder andere Metalloproteasen; Serinproteasen, wie Trypsin, Chymotrypsin, Plasmin etc.; Cysteinproteasen; Aspartat-Proteasen), Elastasen, Hyaluronidasen, Lipasen oder anderer Collagenasen für die Qualität des Verdaus von Vorteil sein.

Die Erfindung wird durch folgende Beispiele, die Figur und das Sequenzprotokoll näher erläutert.

In den Sequenzprotokollen bedeuten:
SEQ ID NO: 1: DNA-Fragment von CHC I
SEQ ID NO: 2: abgeleitetes Proteinfragment
SEQ ID NO: 3: N-terminale Verlängerung
SEQ ID NO: 4: 4 - 24: Primer und Peptidfragmente

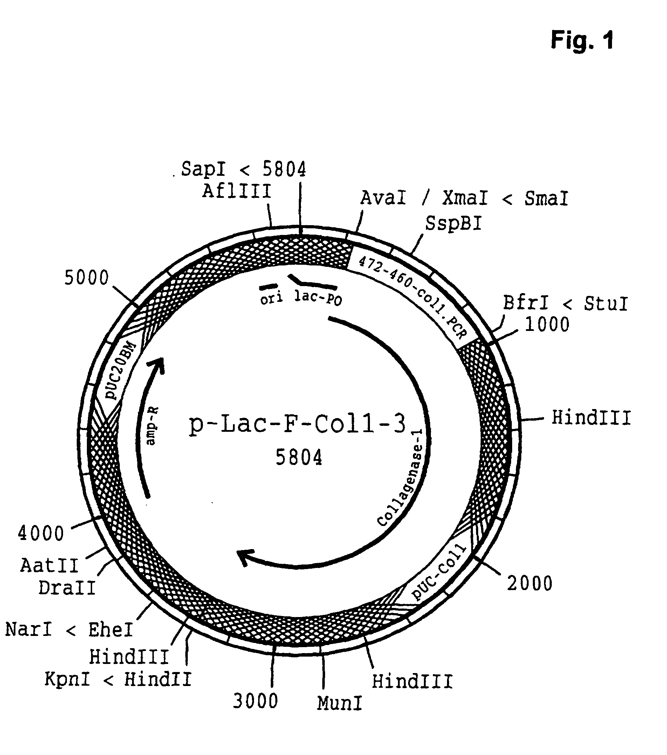

Fig. 1 zeigt den Expressionsvektor p-Lac-F-Coll-3

### Beispiel 1

### Reinigung der Collagenase Class I (CHC I)

1 g Collagenase P (aus Clostridium histolyticum, Boehringer Mannheim GmbH, Kat.-Nr. 1 213 857) wurde in 20 ml H₂O (Milli Q-Qualität) gelöst und partikuläre Bestandteile durch Zentrifugation abgetrennt. Nach 60% Ammoniumsulfat-Fällung wurde der Niederschlag in 9.5 ml H₂O aufgenommen und über Nacht bei 4°C gegen 10 mM Tris-HCl, pH 7,5 dialysiert.

Das Dialysat wurde bei Raumtemperatur auf eine Zn-Chelat-Sepharose-Säule (Säulenvolumen SV = 50 ml, 2 cm Durchmesser) aufgetragen. Anschließend wurde die Säule mit 10 mM Tris HCl, pH 7,5, gewaschen.

Der Durchlaufpeak, der die CHC 1-Fraktionen enthält, wurde über eine Mono Q-Säule (Pharmacia) in 10 mM/l Tris HCl, pH 7,5, unter Verwendung eines linearen Gradienten bis 10 mM/l Tris, pH 7,5, 0,3 M/l NaCl weiter aufgetrennt. Der CHC I-Aktivität enthaltende Peak (Aktivität als Azocoll-Aktivität bestimmt, nach Chavira et al., Anal. Biochem. 136 (1984) 446 - 450) wird über die Reversed-Phase-HPLC weiter gereinigt (Laufmittel A: 0,12 % TFA (Trifluoressigsäure/H₂O, 25 % Acetonitril, Stufengradient bis 50 % Acetonitril in 5 min und bis 70 % Acetonitril in 15 min). Die den Hauptpeak enthaltende Fraktion der zweiten Gradientenstufe wird zur Trockene eingeengt und nach Carboxymethylierung einem Verdau mit Endoproteinase LysC unterworfen. Die Trennung der Peptide erfolgte ebenfalls über Reversed-Phase-HPLC (Vydac C₄) mit einem Gradienten von 5 - 45 % Acetonitril/0,12 % TFA in 105 min. Die Peptide wurden gesammelt, zur Trockene eingeengt und sequenziert.

### Beispiel 2

### Klonierung der Collagenase Class I aus Clostridium histolyticum

**2.1** Abgeleitet von Peptidsequenzen, die durch den Verdau von gereinigter Collagenase I aus Clostridium histolyticum gem. Beispiel 1 gewonnen wurden, wurde mit Hilfe einer PCR-Reaktion ein 1,4 kB Stück des CHC I Gens isoliert. Dieses Stück, bzw. Fragmente davon, wurden zum Herstellen einer DNA-Sonde, die z.B. mit Dig markiert werden kann, verwendet. Eine solche Sonde dient zum Screenen von Genbanken, um den Rest des CHC I Gens zu isolieren.

### Peptidsequenzen:

### Davon abgeleitete Primer:

Es wurden vier Primer entworfen und in zwei verschiedenen PCR-Reaktionen eingesetzt (Primer-Kombinationen 2QF/72R und 2QR/72F). Nur die Kombination 2QF mit 72 R ergab ein PCR-Produkt (Länge 1,4 kB), das der Sequenz von Position 2418 bis 3806 der in SEQ ID NO: 1 angegebenen Gesamtsequenz entsprach.

Die Identität dieses Stücks und des darauf liegenden Leserahmens wurde durch die Wiederauffindung weiterer Peptidsequenzen der Collagenase I aus Clostridium histolyticum erhärtet:

### 2.2 Genbank-Screening:

Aus einer Genbank, die aus Stu I / Hind II geschnittener genomischer DNA von Clostridium histolyticum hergestellt wurde, konnte mit Hilfe eines Digoxigenin-markierten Fragments (abgeleitet von obigem 1,4 kB Fragment) ein DNA Fragment von 5,4 kB Länge isoliert werden, das die gesamte 1,4 kB Sonde enthielt.
Durch DNA-Sequenzierung von den Enden der bekannten 1,4 kB Sequenz, konnte die CHC I Sequenz am 3'-Ende vervollständigt, und am 5'-Ende bis zu einer Stu I Schnittstelle ergänzt werden.

Die Sequenz am 3'-Ende entspricht Position 3807 bis 4358 der in SEQ ID NO:1 angegebenen Gesamtsequenz:

In diesem Sequenzabschnitt konnte die Sequenz eines weiteren Peptides des Wildtyp-Enzyms wiedergefunden werden:

Dieses Peptid überlappt zu einem großen Teil mit Peptid 1-72 (SEQ ID NO:5), enthält aber vier zusätzliche Aminosäuren, die auf dem ursprünglichen 1,4 kB DNA-Fragment noch nicht auffindbar sind.

Die Sequenz am 5'-Ende entspricht Position 1969 bis 2417 der in SEQ ID NO:1 angegebenen Gesamtsequenz.

Aus einer Bgl II Genbank konnte ebenfalls mit der markierten 1,4 kB Sonde ein DNA Fragment von 4,6 kB identifiziert werden. Dieses Fragment erwies sich als ganzes nicht klonierbar, konnte jedoch von der Stu I Site her ansequenziert werden und ergab weitere Sequenzinformationen über den 5' Bereich.

Die gefundene Sequenz entspricht Position 1630 bis 1974 der in SEQ ID NO: 1 angegebenen Gesamtsequenz.

Die Zugehörigkeit dieser Sequenz zum Collagenase I Gen wurde durch das Auffinden eines weiteren Peptides bestätigt:

### 2.3 Inverse PCR:

In einem Versuch, den 5' Bereich des Gens über einen inversen PCR-Ansatz zu fassen, wurde StuI geschnittene genomische DNA aus Clostridium histolyticum im Agarosegel aufgetrennt, der Bereich von 1,9 - 2,7 kB wurde isoliert (aus dem Gel ausgeschnitten), religiert und via inverse PCR amplifiziert:

### Verwendete Primer:

Es wurde ein Fragment von 1,85 kB erhalten, das aber nicht als Ganzes klonierbar war, eine Teilsequenz konnte jedoch ermittelt werden.
Es handelt sich um Position 1384 bis Position 1570 der in SEQ ID NO:1 agegebenen Gesamtsequenz.

Diese Sequenz liegt in der Startregion und unmittelbar upstream der bisher beschriebenen Sequenz für das Collagenase I Gen von Clostridium histolyticum.

### 2.4 Klonierung des 5'-Bereichs des Collagenase I Gens aus Clostridium histolyticum:

Genomische DNA von Clostridium histolyticum wurde mit HindIII/StuI verdaut und im Agarosegel aufgetrennt. Der Bereich zwischen 1,7 und 2,2 kB wurde ausgeschnitten (nach Southern blot erwartetes Fragment liegt bei 1,9 kB). Die DNA wurde aus dem Gel nach konventionellen Methoden isoliert (QiaEx) und in pUC 19 via Hind III / Hind II kloniert.

### 2.5 Genbank-Screening:

Die HindIII/StuI Genbank wurde gescreent mit einer ca. 400 bp großen Dig-markierten Sonde.
Die Sequenz der Sonde entspricht Position 1453 bis 1852 der in SEQ ID NO: 1 angegebenen Gesamtsequenz.

Die Sonde wurde mit Hilfe zweier Primer hergestellt:

Unter ca. 2200 gescreenten Klonen fanden sich 14 mit positivem Signal, davon wurden nach gängigen Methoden Plasmid-Minipreps durchgeführt, die besten 4 Klone enthielten alle dasselbe Insert, welches der Position 1 - 1971 der in SEQ ID NO: 1 angegebenen Gesamtsequenz entspricht.

Mit dieser Sequenz ergibt sich eine Gesamtlänge für das CHC I Gen und seine Upstream Region von ca. 4,4 kB. Die vollständige Sequenz ist in SEQ ID NO: 1 wiedergegeben.

Auf dieser Sequenz liegt ein großer offener Leseraster, der im 3'-Bereich in den bereits beschriebenen Teil der Collagenase I mündet.
Potentielle Startpunkte für die Translation befinden sich an Position 1002 (ATG), 1029 (ATG), 1110 (ATG), sowie 1176 (GTG) und 1254 (GTG).

### 2.6. Im 5'-Bereich verlängerte Konstrukte:

Ein durch Proteinsequenzierung bestimmter N-Terminus einer möglichen reifen Form der Collagenase ( IANTNSEK - SEQ ID NO: 19) liegt bei Position 1332 von SEQ ID NO: 1. Das entsprechende Polypeptid ist in SEQ ID NO:2 wiedergegeben. Da auch alle oben beschriebenen Translations-Startpunkte noch weiter 5' und im Raster liegen, wurden mehrere Expressions-Konstrukte hergestellt, und ihr Expressionsverhalten untersucht.Überraschenderweise zeigt es sich, daß Starts an mehreren Positionen jeweils zu aktiv exprimiertem Enzym fuhren.

### Beispiele für solche Konstrukte:

Durch geeignete Wahl der Primer kann jeweils eine SmaI bzw. AvaI Schnittstelle 5' vor ATG 1002, 1029, 1110 oder auch GTG 1176 oder 1254 eingeführt werden. Verwendet man einen Gegenprimer der jenseits der BfrI Schnittstelle (1965 - 1970) liegt, so können aus einem pUCBM20-Expressionsvektor alle weiteren durch Austausch über SmaI bzw. AvaI und BfrI Schnitt hergestellt werden.

Die unterstrichene Sequenz ist jeweils ein konstruktionsbedingter Fusionsanteil aus pUCBM20.
Es ist bekannt, daß auch GTG-Sequenzen unter Umständen als Startposition in Frage kommen. Ein besonders bevorzugter Start ist bei GTG 1176, das durch Wahl eines geeigneten Primers in ein ATG verwandelt werden kann:

Dies führt zu Expressionsvektor placF-coll-3 (Fig. 1).

### 2.7 Weitere Expressionsmöglichkeiten:

Die bisher beschriebenen Expressionskonstrukte verwenden den lac Promoter und das homologe Signalpeptid. Ebenso gut können statt dessen andere Promotoren eingesetzt werden, wie mgl (EP-B 0 285 152) oder tac (EP-B 0 067 540).
Das Signalpeptid kann auch durch andere, z.B. das mgl-Signalpeptid, ersetzt werden. Die Einsetzung eines solchen heterologen Signalpeptids sollte bevorzugt direkt vor dem reifen 5'-Ende des Collagenase-Gens erfolgen. Es zeigt sich aber auch hier, daß mehrere Positionen denkbar sind. So führt sowohl ein mgl-Signal vor Peptidsequenz IANTNSEK (SEQ ID NO:19) als auch vor AYLTMNTS (SEQ ID NO:24) zu aktiver Expression der CHC I. Der konstruktionsbedingte Fusionsanteil aus pUCBM20 ist ebenfalls nicht erforderlich. Expression erfolgt auch, wenn dieser Fusionsanteil verkürzt oder ganz entfernt wird. Denkbar ist auch die völlige Entfernung des Signals und die cytoplasmatische Expression der CHC I.

### 2.8 Stämme:

Zur Expression der CHC I dienen bevorzugt bekannte E. coli K12-Stämme, wie z.B. C600, HB101 und ähnliche. Andere prokaryontische Systeme, auch grampositive (z.B. Bacillus oder Streptomyces) sowie Expression in eukaryontischen Zellsystemen sind ebenfalls denkbar.

### 2.9 Molekulargewicht:

| | |
|---|---|
| gereinigt (Wildtyp) | ca. 115 kDa (nach SDS-Gelelektrophorese) |
| gereinigt (rec) | ca. 115 kDa (nach SDS-Gelelektrophorese) |
| theoret. (IANTNSEK) | 113897 |
| theoret. (AYLTMNTS) | 122547 |

### Beispiel 3

### Expression von Collagenase Class I

### 10 l Fermentation des E.coli Expressionsklons für CHC I und Hochdruckaufschluß

Aus Stammkulturen (Plattenausstrich, Ampullen oder Pailletten) werden Vorkulturen angesetzt, die geschüttelt bei 30 - 37°C inkubiert werden.
Das Überimpfungsvolumen in die nächsthöhere Dimension beträgt jeweils 1 - 10 Vol%.
Zur Selektion gegen Plasmidverlust wird in Vor- und Hauptkultur Ampicillin (50 - 100 mg/l) eingesetzt.

Als Nährstoffe können enzymatisch verdautes Eiweiß und/oder Hefeextrakt als N- und C-Quelle sowie Glycerin und/oder Glucose als zusätzliche C-Quelle verwendet werden. Das Medium wird auf pH 7 gepuffert, und Metallsalze können zur Stabilisierung des Fermentationsprozesses in physiologisch verträglichen Konzentrationen zugesetzt werden.
Die Fermentation wird als Fedbatch mit einer gemischten Eiweiß/Hefeextrakt Dosage durchgeführt. Durch die Dosagegeschwindigkeit wird die Wachstumsgeschwindigkeit geregelt und mittels Glycerin und/oder Glucose als Säureregulans wird der pH-Wert gesteuert. Über Belüftungsrate, Drehzahlregulierung und Dosagegeschwindigkeit wird der Gelöst-Sauerstoffpartialdruck (pO₂)>20 % gehalten, um anaerobe Bedingungen zu vermeiden. Die Fermentationstemperatur beträgt 25 - 37°C.

Das Wachstum wird über Ermittlung der optischen Dichte bei 528 nm bestimmt. Mittels IPTG oder Lactose wird die Expression der CHC I induziert. Nach einer Fermentationsdauer von ca. 30 - 40 Std. wird bei OD-Stillstand die Biomasse durch Zentrifugation geerntet.

Die Biomasse wird in einem geeigneten Puffer aufgenommen und über eine kontinuierliche Hochdruckpresse bei 1000 Bar aufgeschlossen. Die so erhaltene Suspension wird erneut abzentrifugiert und der Überstand, der die gelöste CHC I enthält, wird weiterverarbeitet.

### Beispiel 4

### Isolierung von Inseln aus Schweine-Pankreas

Aus einem frisch geschlachteten Schwein wird der Pankreas präpariert und in eiskaltem HBSS-Puffer (Gibco) bis zur weiteren Verarbeitung gekühlt. In den Ductus pancreaticus wird eine Braunüle eingeführt und befestigt, die Dichtigkeit des Pankreas wird mit HBSS-Puffer geprüft. Eine Enzymlösung in HBSS-Puffer + Ca²⁺, die die gereinigte rekombinante Collagenase Typ I allein oder in Verbindung mit einer gereinigten nativen oder rekombinanten neutralen Protease und/oder Collagenase Typ II aus Clostridium histolyticum enthält, wird eingespritzt. Der so behandelte Pankreas wird an die ebenfalls obige Enzymlösung enthaltende Perfusionseinheit (diskontinuierliche Perfusion) angeschlossen. Der Verdau erfolgt zwischen 4°C - 37°C in einem Zeitraum von 5 bis 120 Minuten. Nach der als optimal angenommenen Zeit wird die Pumpe gestoppt und das den Pankreas enthaltende Gefäß 3 bis 20 Minuten lang vorsichtig per Hand geschüttelt. Im Bedarfsfall vorher zugegebene Metallkugeln erleichtern zusätzlich die mechanische Dissoziation des Gewebes und die Freisetzung der Inseln aus dem umgebenden exokrinen Gewebe.

Der Verdau wird durch Zugabe von eiskaltem HBSS/10 % FKS (fötales Kälberserum) gestoppt und die Suspension durch ein Sieb (Maschengröße 300 µm) filtriert, um die Grobteile abzutrennen. Die im Durchfluß befindlichen Inseln werden 10 Minuten bei 100 g in 250 ml Naigene-Rundbodenflaschen abzentrifugiert. Der Überstand wird abgenommen und das die Inseln enthaltende Pellet in 50 ml FKS resuspendiert.

Eine weitere Aufreinigung der Inseln kann über einen per Hand hergestellten Dichtegradienten erfolgen. In 250 ml Nalgene-Rundbodenflaschen werden zunächst 7 ml der Inselsuspension gegeben. Diese wird zunächst mit 93 ml einer Ficoll-Lösung® (ϕ = 1.077 g/cm³), darauffolgend mit 50 ml Medium (RPMI 1640) überschichtet. Diese Gradienten werden im Ausschwingrotor bei 100 g für 10 Minuten zentrifugiert. Fraktionen á 10 ml werden abgenommen, die Größe, Reinheit und Ausbeute der mit Dithizon gefärbten Inseln wird in jeder Fraktion mikroskopisch oder mit Hilfe der Bildanalytik bestimmt.

### Referenzliste

Amann et al., Gene 40 (1985) 183
Angleton, E.L. und Van Wart, H.E., Biochemistry 27 (1988) 7413 - 7418 und 7406 - 7412
Beck und Zink, Gene 16 (1981) 35-58
Bond, M.D. und Van Wart, H.E., Biochemistry 23 (1984) 3077 - 3085
Bond, M.D. und Van Wart, H.E., Biochemistry 23 (1984) 3085 - 3091
Brosius et al., J. Mol. Biol. 148 (1981) 107 - 127
Chang et al., Nature 198 (1977) 1056
Davies et al., Ann. Rev. Microbiol. 32 (1978) 469
EP-A 0 267 851
EP-A 0 495 398
French, M.F., et al., J. Protein Chemistry 11 (1992) 83 - 97
Ghrayeb et al., EMBO J. 3 (1984) 2437
Goeddel et al., Nuc. Acids Res. 8 (1980) 4057
Hefley et al., J. Bone Mineral Res. 2 (1987) 505 - 516
Holzinger et al., Immunol. Lett. 35 (1993) 109 - 118
Kingsman, S.M., et al, Tibtech 5 (1987) 53- 57
Miller et al., Bio/Technology 7 (1989) 698
Nagai et al., Nature 309 (1984) 810
Oka et al., Proc. Natl. Acad. Sci. USA 82 (1985) 7212
Palva et al., Proc. Natl. Acad. Sci. USA 79 (1982) 5582
Phillips and Silhavy, Nature 344 (1990) 882 - 884
Powell et al., Appl. Environ. Microbiol. 54 (1988) 655
Quibel et al., Anal. Biochem. 154 (1986) 26 - 28
Ricordi et al., Diabetes 37 (1988) 413 - 420
Sambrook et al., "Expression of cloned genes in E. coli" in Molecular Cloning: A laboratory manual (1989) Cold Spring Harbor Laboratory Press, New York, USA
Shimatake et al., Nature 292 (1981) 128
Studier et al., J. Mol. Biol. 189 (1986) 113
Sutton et al., Transplantation 42 (1986) 689 - 691
US-Patent 4,551,433
US-Patent 4,689,406
US-Patent 4,747,056
US-Patent 5,177,011
US-Patent 5,418,157
Van Wart, H.E., Biochemistry 24 (1985) 6520 - 6526
WO 91/11520
WO 91/14447
WO 94/00580
Wolters, Hormone and Metabolic Research 26 (1994) p. 80
Yansura, Meth. Enzymol. 185 (1990) 161 - 166

## Patentansprüche

1. Polypeptid mit den Eigenschaften einer Collagenase Class I aus Clostridium histolyticum (CHC I) und der Aminosäuresequenz SEQ ID NO:2, oder SEQ ID NO:2 welche N-terminal um eine oder mehrere Aminosäuren der Sequenz SEQ ID NO:3 verlängert ist.

2. Polypeptid nach Anspruch 1, für welches die codierende Nukleinsäure mit Nukleotid 1002, 1029, 1110, 1176 oder 1254 von SEQ ID NO: 1 beginnt.

3. Nukleinsäure, welche für ein Polypeptid mit den Eigenschaften einer Collagenase Class I aus Clostridium histolyticum (CHC I) mit SEQ ID NO:2, oder SEQ ID NO:2 welche um eine oder mehrere Aminosäuren von SEQ ID NO:3 am N-Terminus verlängert ist, codiert.

4. Nukleinsäure nach Anspruch 3, welche mit Nukleotid 1002, 1029, 1110, 1176 oder 1254 von SEQ ID NO:1 1 beginnt.

5. Verfahren zur Herstellung eines Polypeptids mit den Eigenschaften einer Collagenase Class I aus Clostridium histolyticum (CHC I) durch Expression einer exogenen Nukleinsäure in prokaryontischen oder eukaryontischen Wirtszellen und Isolierung des gewünschten Peptids, wobei die exogene Nukleinsäure für ein Polypeptid mit der SEQ ID NO:2 oder für ein Polypeptid gemäß SEQ ID NO:2, welches N-terminal um eine oder mehrere Aminosäuren der SEQ ID NO:3 verlängert ist, codiert.

6. Verfahren nach Anspruch 5, **dadurch gekennzeichnet, daß** die exogene Nukleinsäure mit Nukleotid 1002, 1029, 1110, 1176 oder 1254 von SEQ ID NO:1 beginnt.

7. Verwendung eines Polypeptids nach den Ansprüchen 1 oder 2 zur Isolierung von Zellen aus Gewebe von Säugetieren und aus Humangewebe.

## Claims

1. Polypeptide having the properties of a class I collagenase from Clostridium histolyticum (CHC I) and the amino acid sequence SEQ ID NO:2 or SEQ ID NO:2 which is N-terminally extended by one or more amino acids of the sequence SEQ ID NO:3.

2. Polypeptide as claimed in claim 1, for which the coding nucleic acid begins with nucleotide 1002, 1029, 1110, 1176 or 1254 of SEQ ID NO:1.

3. Nucleic acid which codes for a polypeptide with the properties of a class I collagenase from Clostridium histolyticum (CHC I) having SEQ ID NO: 2 or SEQ ID NO:2 which is extended at the N-terminus by one or more amino acids of SEQ ID NO:3.

4. Nucleic acid as claimed in claim 3 which begins with nucleotide 1002, 1029, 1110, 1176 or 1254 of SEQ ID NO: 1.

5. Process for the production of a polypeptide having the properties of a class I collagenase from Clostridium histolyticum (CHC I) by expression of an exogenous nucleic acid in prokaryotic or eukaryotic host cells and isolation of the desired peptide, wherein the exogenous nucleic acid codes for a polypeptide having SEQ ID NO:2 or for a polypeptide of SEQ ID NO:2 which is extended N-terminally by one or more amino acids of sequence SEQ ID NO:3.

6. Process as claimed in claim 3, wherein the exogenous nucleic acid begins with nucleotide 1002, 1029, 1110, 1176 or 1254 of SEQ ID NO:1.

7. Use of a polypeptide as claimed in claims 1 or 2 for the isolation of cells from mammalian tissue and human tissue.

## Revendications

1. Polypeptide ayant les propriétés d'une collagénase de classe I issue de *clostridium histolyticum* (CHC I) et de la séquence d'acides aminés SEQ ID NO:2, ou SEQ ID NO:2 qui est prolongée au niveau du site N-terminal par un ou plusieurs acides aminés de la séquence SEQ ID NO:3.

2. Polypeptide selon la revendication 1, pour lequel l'acide nucléique codant commence par le nucléotide 1002, 1029, 1110, 1176 ou 1254 de la séquence SEQ ID NO:1.

3. Acide nucléique qui code pour un polypeptide ayant les propriétés d'une collagénase de classe I issue de *clostridium histolyticum* (CHC I) avec la SEQ ID NO:2, ou SEQ ID NO:2 qui est prolongée au niveau du site N-terminal par un ou plusieurs acides aminés de la séquence SEQ ID NO:3.

4. Acide nucléique selon la revendication 3, qui commence par le nucléotide 1002, 1029, 1110, 1176 ou 1254 de la séquence SEQ ID NO:1.

5. Procédé de préparation d'un polypeptide ayant les propriétés d'une collagénase de classe I issue de *clostridium histolyticum* (CHC I) par expression d'un acide nucléique exogène dans des cellules hôtes procaryotes ou eucaryotes et par isolation du peptide souhaité, l'acide nucléique exogène codant pour un polypeptide ayant la séquence SEQ ID NO:2 ou pour un polypeptide selon la séquence SEQ ID NO:2, qui est prolongée au niveau du site N-terminal par un ou plusieurs acides aminés de la séquence SEQ ID NO:3.

6. Procédé selon la revendication 5, **caractérisé en ce que** l'acide nucléique exogène commence par le nucléotide 1002, 1029, 1110, 1176 ou 1254 de la séquence SEQ ID NO:1.

7. Utilisation d'un polypeptide selon les revendications 1 ou 2, pour isoler des cellules provenant de tissu de mammifères et de tissu humain.
